(19) Europäisches Patentamt
European Patent Office
Office européen des brevets

(11) **EP 1 792 562 A1**

(12) **EUROPEAN PATENT APPLICATION**

(43) Date of publication:
**06.06.2007 Bulletin 2007/23**

(51) Int Cl.:
*A61B 3/107* (2006.01)  *A61F 2/14* (2006.01)

(21) Application number: **06256136.0**

(22) Date of filing: **30.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **01.12.2005 US 293644**

(71) Applicant: **Revision Optics, Inc.**
**Lake Forest, CA 92630 (US)**

(72) Inventors:
• **Lang, Alan**
**Long Beach,**
**California 90807 (US)**
• **Miller, Troy**
**Rancho Santa Margarita,**
**California 92688 (US)**

(74) Representative: **Woods, Geoffrey Corlett**
**J.A. KEMP & CO.**
**Gray's Inn**
**14 South Square**
**London WC1R 5JJ (GB)**

(54) **Design of intracorneal inlays**

(57) Described herein are designs of intracorneal inlays to produce desired shapes of the cornea's anterior surface for correcting vision impairments. In an exemplarily embodiment, a thickness profile is defined by a difference between a desired post-operative anterior corneal surface that corrects a patient's vision impairment and the patient's pre-operative anterior corneal surface. An inlay is then dimensioned to substantially have the same thickness profile. When implanted in the patient's cornea, the thickness profile of the inlay is substantially transferred to the anterior corneal surface through the intervening corneal tissue, thereby producing the desired post-operative anterior corneal surface.

EP 1 792 562 A1

**Description**

FIELD OF THE INVENTION

The field of the invention relates generally to corneal implants, and more particularly, to intracorneal inlays.

BACKGROUND INFORMATION

As is well known, abnormalities in the human eye can lead to vision impairment. Some typical abnormalities include variations in the shape of the eye, which can lead to myopia (nearsightedness), hyperopia (far-sightedness) and astigmatism as well as variations in the tissue present throughout the eye, such as a reduction in the elasticity of the lens, which can lead to presbyopia. Corneal implants have been used successfully to treat these and other types of vision impairment.

Corneal implants typically correct vision impairment by altering the shape of the cornea. Corneal implants can be classified as an onlay and an inlay. An onlay is an implant that is placed over the cornea such that the outer layer of the cornea, e.g., the epithelium, can grow over and encompass the implant. An inlay is an implant that is surgically implanted into the cornea beneath a portion of the corneal tissue by, for example, cutting a flap in the cornea and inserting the inlay beneath the flap. The inlay in the cornea alters the shape of the cornea's anterior surface, and therefore the refractive power of the cornea. Since the cornea is the strongest refracting optical element in the human ocular system, altering the cornea's anterior surface is a particularly useful method for correcting vision impairments caused by refractive errors. Inlays are also useful for correcting other visual impairments including presbyopia.

An essential step in using an inlay to correct vision impairment is designing the inlay to produce a desired shape of the anterior corneal surface, which requires accurately modeling the effect of the inlay's geometry on the shape of the anterior corneal surface. One model was proposed in Warsky, M., el al, "Predicting Refractive Alterations with Hydrogel Keratophakia", Investigative Ophthalmology & Visual Science, Vol 26, No 2, 1985, pp 240-243. In this model ("Warsky model"), the predicted radius of curvature of the anterior corneal surface is equal to the sum of the radius of curvature of the inlay and the thickness of the overlaying flap. However, in clinical tests, the Warsky model did not accurately predict the actual post-operative refractive outcome of inlay implantation in the correction of hyperopia. This suggests that the Warsky model does not provide a good basis for the design of intracorneal inlays.

Accordingly, there is a need for improved designs of intracorneal inlays that more accurately produce desired shapes of the anterior corneal surface for correcting vision impairments.

SUMMARY

The present invention provides improved designs of intracorneal inlays to produce desired shapes of the anterior corneal surface for correcting vision impairments.

In an exemplarily embodiment, an inlay is designed for a patient experiencing vision impairment. First, a desired post-operative anterior corneal surface that corrects the patient's vision impairment is determined. A thickness profile is then defined by a difference between the desired post-operative anterior corneal surface and the patient's pre-operative anterior corneal surface. The inlay is then dimensioned to substantially have the same thickness profile. When implanted in the patient's cornea, the thickness profile of the inlay is substantially transferred to the anterior corneal surface through the intervening corneal tissue, thereby producing the desired post-operative anterior corneal surface.

The inlay designs according to the invention are useful for correcting a wide range of vision impairments by varying the shape of the desired anterior corneal surface used to define the thickness profile of the inlay. For example, the desired anterior corneal surface can be spherical to correct refractive errors due to, for example, hyperopia or myopia. For another example, the desired anterior corneal surface can be aspheric to correct refractive errors and higher-order aberrations of the patient's ocular system. For yet another example, the desired corneal surface can form a two-zone, bifocal simultaneous vision, surface to correct both prebyopes and hyperopia. The desired anterior corneal surface can be of any other shape for the correction of other vision impairments.

Other systems, methods, features and advantages of the invention will be or will become apparent to one with skill in the art upon examination of the following figures and detailed description. It is intended that all such additional systems, methods, features and advantages be included within this description, be within the scope of the invention, and be protected by the accompanying claims. It is also intended that the invention not be limited to the details of the example embodiments.

BRIEF DESCRIPTION OF THE FIGURES

Figure 1 is a cross-sectional view of a cornea showing a thickness profile according to an embodiment of the invention.

Figure 2 is a cross-sectional view of the cornea of Figure 1 rotated 90 degrees counterclockwise.

Figure 3 is a cross-sectional view of an anterior corneal surface forming a two-zone, bifocal simultaneous vision, surface according to an embodiment of the invention.

Figure 4 is a cross-sectional view of an aspheric anterior corneal surface according to an embodiment of the invention.

Figure 5a is a cross-sectional view of a cornea with a flap cut into the cornea using an intralase keratome.

Figure 5b is a cross-sectional view of a cornea with a flap cut into the cornea using a mechanical keratome.

Figure 6a is a cross-sectional view of the cornea of Figure 3a with an inlay according to an embodiment of the invention implanted beneath the flap.

Figure 6b is a cross-sectional view of the cornea of Figure 3b with an inlay according to an embodiment of the invention implanted beneath the flap.

## DETAILED DESCRIPTION

Described herein are designs of intracorneal inlays to produce desired shapes of the anterior corneal surface for correcting vision impairments.

A first step in the design of an inlay in accordance with the invention is determining a profile thickness that the inlay must induce on the anterior corneal surface to produce a desired anterior corneal surface. Although not so limited, the thickness profile may be illustrated by way of an example. In this example, a thickness profile is specified for the correction of hyperopia (far-sightedness). Hyperopia occurs when light entering the eye focuses behind the retina. Hyperopia can be corrected by increasing the curvature of the anterior corneal surface and thereby the refractive power of the cornea, which causes light to bend more in the cornea and focus at the retina. This type of correction is known as hyperopic correction.

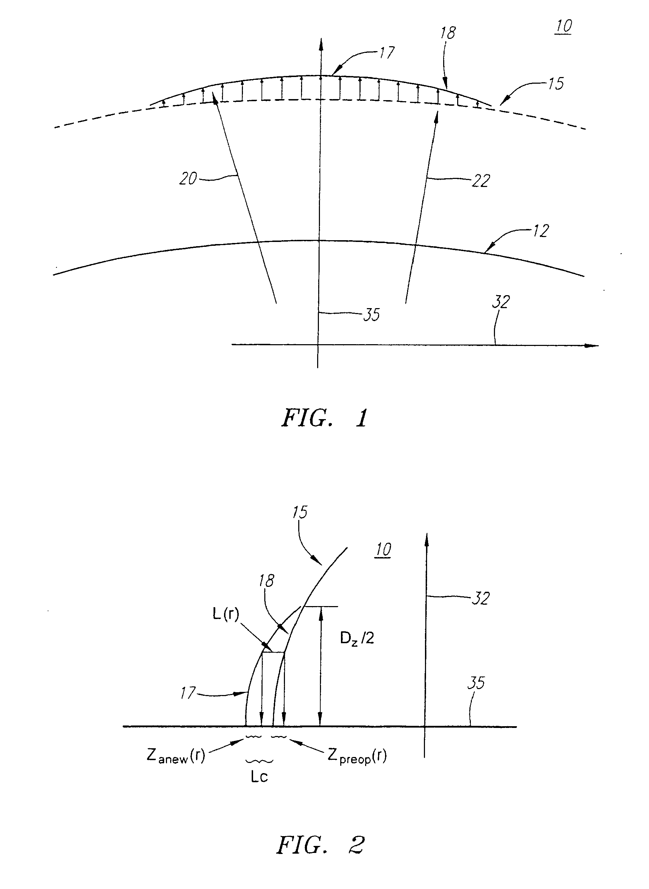

The objective in this example is to increase the curvature of the cornea's anterior surface to achieve the desired hyperoptic correction. Figure 1 shows a cross-sectional view of a cornea 10. The cornea 10 comprises a posterior portion 12 and a pre-operative anterior surface 15. The pre-operative anterior surface 15 is the anterior surface of the cornea before implantation of the inlay. Also shown is a desired anterior surface 17 of the cornea 10 that provides the necessary hyperoptic correction.

A first step is to determine an anterior radius of curvature, $r'_a$, that provides the desired refractive change, $\Delta Rx$ = Rxpre - Target, where Rxpre is a pre-operative spherical equivalent of the cornea's refractive power and Target is a targeted post-operative equivalent of the cornea's refractive power. The equivalent change in the cornea's refractive power, $\Delta K_{equiv}$, at the anterior surface is given by:

$$\Delta K_{equiv} = \frac{1}{\frac{1}{Rxpre} - V} - \frac{1}{\frac{1}{Target} - V} \qquad \text{Equation 1}$$

where V is a spectacle vertex distance, e.g., 0.012 meters, from a spectacle to the cornea's anterior surface. The spectacle vertex distance, V, takes into account that measurements of the cornea's refractive power are typically taken with a spectacle located a distance from the cornea's anterior surface, and translates these power measurements to the equivalent power at the cornea's anterior surface.

The pre-operative refractive power at the anterior corneal surface may be approximated by Kavg - Kpost, where Kavg is the average corneal refractive power and Kpost is a posterior corneal refractive power. The desired radius of curvature, $r'_a$, of the anterior surface may be given by:

$$r'_a = \frac{(1.376-1)}{(Kavg - Kpost + \Delta K_{equiv})} \qquad \text{Equation 2}$$

For purposes of design and analysis, Kpost may be approximated as -6 diopters. The pre-operative radius of curvature, $r_{preop}$, may be approximated by:

$$r_{preop} = (1.376\text{-}1) / (Kavg - Kpost) \qquad \text{Equation 3}$$

In Figure 1, the desired radius of curvature, $r'_a$, is show by line 20 and pre-operative radius of curvature, $r_{preop}$, is shown by line 22. The two radius of curvatures need not originate from the same origin.

Figure 1 shows a cross-sectional view of a thickness profile 18 specified by a difference between the desired anterior corneal surface 17 and the pre-operative anterior corneal surface 15. In Figure 1, arrows pointing from the pre-operative anterior surface 15 to the desired anterior surface 17 represent the axial thickness, L(r), of the thickness profile 18 at different positions along an r axis 32 that is substantially perpendicular to an optical z axis 35. The double arrow represents a center thickness, $L_c$, of the thickness profile 18. In this embodiment, the thickness profile 18 is rotationally symmetric about the z axis 35. Thus, the entire thickness profile 18 may be defined by rotating the cross-sectional view shown in Figure 1 about the z axis 35.

Figure 2 shows a cross-sectional view of the cornea 10 rotated counterclockwise 90 degrees relative to Figure 1. The thickness L(r) of the thickness profile 18 may be given by:

$$L(r) = L_c + Z_{preop}(r) - Z_{anew}(r) \quad \text{and} \quad L_c = Z_{anew}\left(\frac{d_z}{2}\right) - Z_{preop}\left(\frac{d_z}{2}\right) \qquad \text{Equation 4}$$

where $L_c$ is the center thickness of the thickness profile 18, $Z_{preop}(r)$ is the pre-operative anterior corneal surface 15 as a function of r, $Z_{anew}(r)$ is the desired anterior corneal surface 17 as a function of r, and $d_z$ is the diameter of the optical zone. The optical zone diameter $d_z$ is the diameter of the post-operative surface 17 which produces acceptable image quality, e.g., 5.0 mm. The optical zone for intracorneal inlays correcting hyperopia may range from about 4 mm to 6 mm in diameter. For the case of hyperoptic correction, $Z_{anew}$ has a radius of curvature equal to $r'_a$, and $Z_{preop}$ has a radius of curvature equal to $r_{preop}$.

In the example above, the anterior surfaces $Z_{anew}$ and $Z_{preop}$ were assumed to be spherical. This need not be the case. The anterior surfaces may also be aspheric. More generally, the desired anterior surface $Z_{anew}$ may be a function of hyperoptic correction and also more complex design parameters, e.g., an aspheric surface for higher-order aberration correction, or a more complex zonal surface for presbyoptic correction. Also, the pre-operative anterior surface $Z_{preop}$ is generally aspheric. For designs requiring aspheric surfaces, the surface function Z(r) may be given by the general aspheric form:

$$Z(r) = \frac{r^2 / r_c}{1 + \sqrt{1 - (1+k)(r / r_c)^2}} + a_4 r^4 + a_6 r^6 \qquad \text{Equation 5}$$

where:

$r_c$ is the radius of curvature
k is a conic constant
$a_4$ and $a_6$ are higher order aspheric constants

For a spherical surface, k = 0, $a_4$ = 0, and $a_6$ = 0. The human cornea may be approximated by k = -0.16, $a_4$ = 0 and $a_6$ = 0. The radius of curvature, $r_c$, may specify correction for refractive error due to hyperopia or myopia, and the other parameters may specify corrections for higher-order aberrations.

The above expressions for the thickness profile are intended to be exemplary only. Other mathematical expressions or parameters may be used to describe similar or other thickness profiles. Therefore, the invention is not limited to particular mathematical expressions or parameters for describing the thickness profile. In addition, the thickness profile may be specified by surfaces of any other shape for the correction of other vision impairments. For example, the thickness profile may be specified by multi-focal surfaces, simultaneous vision surfaces, multi-zonal surfaces, and the like.

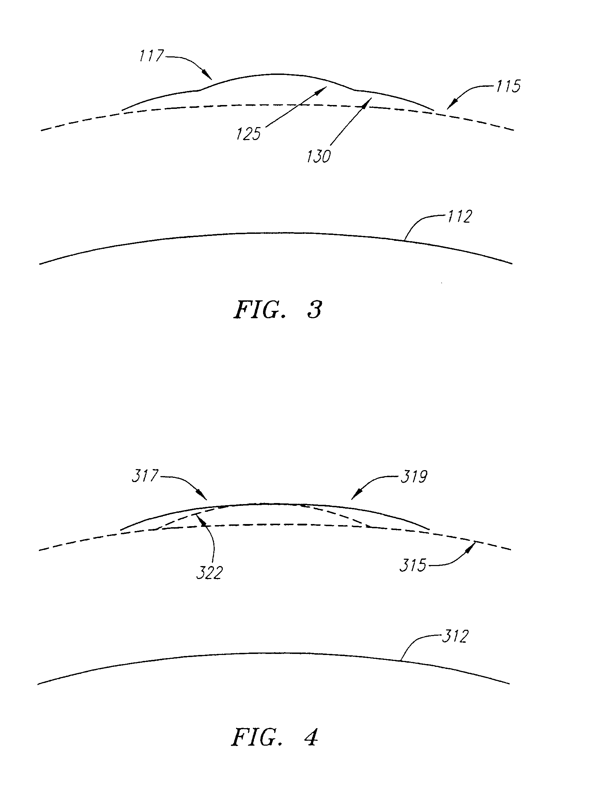

For example, Figure 3 is a cross-sectional view of an anterior corneal surface 117 forming a two-zone, bifocal simultaneous vision, surface. The central zone 125 has a higher curvature, providing optical power to focus near objects for patients lacking natural accommodation (e.g., presbyopes). The outer zone 130 provides the necessary diopter power to correct hyperopic refractive power. Also shown is the pre-operative anterior surface 115 and the posterior 112 of the cornea.

For another example, Figure 4 is a cross-sectional view of an anterior corneal surface 317, whose flatter profile 319 allows for the correction of both hyperopic refractive error and positive spherical aberration of the patient's ocular system. Also shown is a spherical surface 322 correcting hyperopic refractive error, the pre-operative anterior surface 315, and the posterior 312 of the cornea.

After the required thickness profile is determined, the inlay is dimensioned to have substantially the same thickness profile, e.g., to within about a micron. The inlay may have a diameter of, e.g., 5 mm, and a center thickness, e.g., in the range of 5 $\mu$m to 100 $\mu$m. When implanted in the cornea, the thickness profile of the inlay is substantially transferred to the anterior corneal surface through the intervening corneal tissue, thereby producing the desired post-operative anterior corneal surface. The transfer of the inlay's thickness profile to the anterior corneal surface may be illustrated by way of examples.

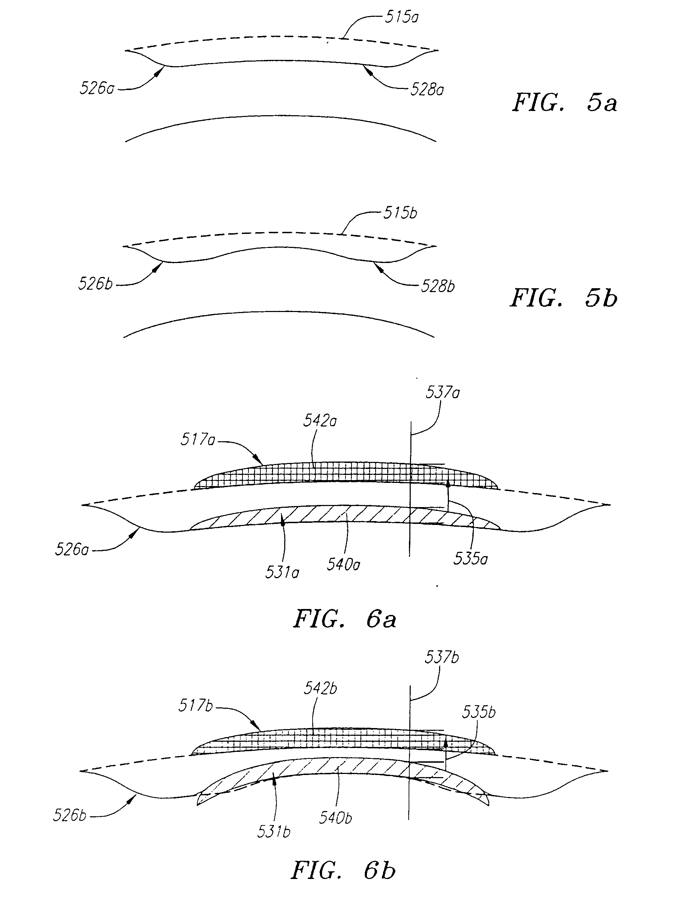

Figures 5a and 5b show cross-sectional views of lamellar flaps 526a and 526b cut into the cornea. The flaps 526a and 526b are used to implant the inlay in the interior, e.g., stroma, of the cornea and may have a depth of, e.g., 100 $\mu$m. The flaps typically remain attached to the cornea by a hinge (not shown). Figure 5a shows a flap 526a cut into the cornea with an intralase keratome, e.g., a laser. The intralase creates a flap bed shape 528a approximately equal to a constant depth from the anterior corneal surface 515a. Figure 5b shows a flap 526b cut into the cornea with a mechanical keratome, e.g., mechanical blade. Based on pachymetry measurements, it is suspected that the mechanical keratome flap 526b is thinner at the corneal apex and deeper at the flap boundaries as shown in Figure 5b.

Figures 6a and 6b show inlays 531a and 531b according to the invention implanted in the cornea of Figures 5a and 5b beneath the respective flaps 526a and 526b. This may be done by lifting the flaps 526a and 526b, placing the inlays 531a and 531b on the exposed area of the cornea's interior, and repositioning the flaps 526a and 526b over the inlays 531a and 531b. In each case, the inlay 531a and 531b contours to the bed shape 528a and 528b lying beneath the respective overlying flap 526a and 526b, and the thickness profile 540a and 540b of the inlay 531 a and 531b displaces the overlying flap material substantially in the optical axial direction, represented by the arrow 535a and 535b. As a result, the thickness profile 540a and 540b of the inlay 531a and 531b is substantially transferred to the anterior corneal surface through the intervening flap material thereby producing the desired anterior corneal surface 517a and 517b. The thickness profile 542a and 542b induced on the anterior corneal surface 517a and 517b by each inlay 531a and 531b is also shown. Although the inlay 531b in Figure 4b has a different shape than the anterior corneal surface 517b, their thickness profiles 540b and 542b are substantially the same. Thus, a line 537b intersecting the two thickness profiles 540b and 542b in the axial direction will measure substantially the same thickness.

The transfer of the inlay's thickness profile to the anterior corneal surface through the flap material may be based on the following assumptions:

a. The inlay thickness profile remains approximately constant as the inlay conforms to the bed shape of the flap when inserted below the flap. This appears reasonable to first order because the inlay is typically more than 70% water (e.g., incompressible) and the forces exerted on the inlay are not great enough to change the inlay hydration. Note that the flap is first order a "free" piece of tissue. The lamella cut during the flap creation do not re-attach in a way to recover the tension along the sclera-to-sclera arc.

b. The inlay conforms to the bed geometry of the flap.

c. The deformation of the anterior corneal surface by the inlay and flap geometries is approximately along the optical axis instead of radially. To radially push the overlying flap volume, the geometry must allow the periphery of the flap to expand radially. The uncut cornea, peripheral to the keratome cut forming the flap, presents a barrier to that increased diameter. Additionally, the corneal stroma (flap material) is composed of layers of lamellar fibers, roughly perpendicular to the optical axis. As such, elastic deformation along the lamellae (e.g., radial expansion) is more difficult than deformation perpendicular to the fibers, suggesting a preferential tendency for axial displacement due to the inlay.

d. The bed shape is proportional to the anterior corneal curvature. Lower power corneas have flatter bed shapes and stronger power corneas have steeper bed shapes, regardless of the flap cut geometry.

The inlay designs of the invention differ from the Warsky model in several respects. First, the inlay designs of the invention assumes that the inlay substantially displaces the overlying flap axially, whereas, the Warksy model implies that the inlay displaces the overlying flap radially. Consequently, the inlay designs of the invention predict that the thickness profile of the inlay is substantially transferred to the anterior cornea surface. In contrast, the Warksy model predicts that the thickness profile induced at the anterior cornea surface expands radially from the thickness profile of the inlay. Also, in the Warsky model, the radius of curvature of the anterior corneal surface due to an inlay depends on flap thickness. The inlay designs of the invention are approximately independent of flap thickness.

In a clinical test, an exemplary inlay design of the invention was compared to an inlay based on the Warsky model. In the test, the inlay design and the Warksy model were used to predict the expected post-operative refractive outcome (Rxpost) for subjects with implants of known geometry and known flap geometry. The standard deviation of the difference between the actual post-operative refractive outcome (Rxactual) and Rxpost was used as a measure of the accuracy.

Table 1 below summarizes the standard deviations of the actual post-operative refractive outcome Rxactual, and the predicted post-operative refractive outcome Rxpost of the inlay design of the invention and the Warksy model. The data is given for mechanical and Intralase keratomes separately, since each creates a different flap geometry. The data below includes optimization of two general constants, attempting to make the Warsky model as accurate as possible. The constants include a coefficient to the inlay's designed anterior radius of curvature to account for potential changes in the inlay's anterior radius of curvature when the inlay conforms to the bed geometry. Another additive constant was added to the clinically measured flap thickness to account for methodological errors in the measurement or consistent offsets to the flap thickness due to biomechanical properties.

Clearly, the inlay design of the invention, based on axial displacement of flap material, achieves the same predictability as the actual results. In contrast, the Warsky model has significantly higher standard deviations, suggesting that the model is less accurate. Consistent with these results, the same standard deviation of clinical data provided in the Warsky paper demonstrate a value of 1.52 diopters, similar the results in Table 1.

Table 1

| Summary of standard deviations from target | | | |
|---|---|---|---|
| | Rxactual | Exemplary Inlay design of invention | Warsky Method |
| Mechanical | 0.64 diopters | 0.62 diopters | 1.01 diopters |
| Intralase | 0.94 diopters | 0.88 diopters | 1.53 diopters |

As a percentage of original postop Rx

| | Exemplary Inlay design of invention | Warsky Method |
|---|---|---|
| Mechanical | 97% | 158 % |
| Intralase | 94 % | 163 % |

In the foregoing specification, the invention has been described with reference to specific embodiments thereof. It will, however, be evident that various modifications and changes may be made thereto without departing from the broader spirit and scope of the invention. For example, the invention does not require that the inlay's thickness profile be exactly the same as the desired thickness profile. The thickness profiles may be substantially the same while differing slightly depending on the precision with which the inlays can be fabricated. In addition, minor adjustments may be made to the inlay's thickness profile to compensate for, e.g., edge thickness of the inlay, without departing from the scope of the invention. As another example, each feature of one embodiment can be mixed and matched with other features shown in other embodiments. As yet another example, the order of steps of method embodiments may be changed. Features and processes known to those of ordinary skill may similarly be incorporated as desired. Additionally and obviously, features may be added or subtracted as desired. Accordingly, the invention is not to be restricted except in light of the attached claims and their equivalents.

**Claims**

1. A method for designing an intracorneal inlay to correct vision impairment of a patient, the patient having a pre-operative anterior corneal surface, comprising:

   determining a desired anterior corneal surface for correcting the vision impairment;
   defining a thickness profile by a difference between the desired anterior corneal surface and the pre-operative anterior corneal surface; and
   dimensioning the inlay to have substantially the thickness profile.

2. The method of claim 1, wherein the desired anterior corneal surface comprises a spherical surface or an aspheric surface.

3. The method of claim 1 or 2, wherein the desired anterior corneal surface comprises a multi-focal surface, a simultaneous vision surface or a multi-zonal surface.

4. The method of any one of the preceding claims, wherein the desired anterior corneal surface has a higher curvature than the pre-operative anterior corneal surface or a lower curvature than the pre-operative anterior corneal surface.

5. The method of any one of the preceding claims, wherein the inlay comprises hygrogel and/or has a diameter in the range of 1.5 mm to 6 mm.

6. The method of any one of the preceding claims, wherein the thickness profile is given by:

$$L(r) = L_c + Z_{preop}(r) - Z_{anew}(r)$$

   where $L(r)$ is the thickness profile as a function of r, $L_c$ is a center thickness of the thickness profile, $Z_{preop}(r)$ is the pre-operative anterior corneal surface as a function of r, $Z_{anew}(r)$ is the desired anterior corneal surface as a function of r, and r is a position along a radial axis substantially perpendicular to an optical axis of the patient's eye.

7. An intracorneal inlay for correcting vision impairment of a patient, wherein the inlay corneal implant is dimensioned to have a thickness profile defined by a difference between a desired anterior corneal surface for correcting the vision impairment and a pre-operative anterior corneal surface of the patient.

8. The inlay of claim 7, wherein the desired anterior corneal surface comprises:

   (a) a spherical surface or an aspheric surface, and/or
   (b) a multi-focal surface, a simultaneous vision surface or a multi-zonal surface.

9. The inlay of claim 7 or 8, wherein the inlay comprises hygrogel and/or has a diameter in the range of 1.5 mm to 6 mm.

10. The inlay of any one of claims 7 to 9, wherein the thickness profile is given by:

$$L(r) = L_c + Z_{preop}(r) - Z_{anew}(r)$$

   where $L(r)$ is the thickness profile as a function of r, $L_c$ is a center thickness of the thickness profile, $Z_{preop}(r)$ is the pre-operative anterior corneal surface as a function of r, $Z_{anew}(r)$ is the desired anterior corneal surface as a function of r, and r is a position along a radial axis substantially perpendicular to an optical axis of the patient's eye.

**FIG. 1**

**FIG. 2**

117

115

125

130

112

**FIG. 3**

317

319

322

315

312

**FIG. 4**

FIG. 5a

FIG. 5b

FIG. 6a

FIG. 6b

**European Patent Office**

## EUROPEAN SEARCH REPORT

Application Number

EP 06 25 6136

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 308 077 A (COOPER CO INC) 22 March 1989 (1989-03-22) * page 2, lines 7-10; claims; figures * * page 4, lines 14-22 * * page 5, line 38 - page 7, line 55 * ----- | 1-10 | INV. A61B3/107 A61F2/14 |
| A | WO 03/041616 A (PEYMAN GHOLAM A) 22 May 2003 (2003-05-22) * paragraphs [0048], [0058]; claims; figures * ----- | 1-10 | |
| A | CHURMS P W: "The theory and computation of optical modifications to the cornea in refractive keratoplasty." AMERICAN JOURNAL OF OPTOMETRY AND PHYSIOLOGICAL OPTICS FEB 1979, vol. 56, no. 2, February 1979 (1979-02), pages 67-74, XP008077152 ISSN: 0093-7002 * the whole document * ----- | 1-10 | |
| A | US 5 716 633 A (CIVERCHIA LINDA) 10 February 1998 (1998-02-10) * column 7, lines 1-5 * * column 8, lines 25-29 * * column 9, lines 40-43 * * column 17, lines 16-18; claims; figure 4 * ----- | 1-10 | TECHNICAL FIELDS SEARCHED (IPC) A61B A61F |
| A | WO 03/101341 A (SCIENT OPTICS INC) 11 December 2003 (2003-12-11) * claims; figures * ----- | 1-10 | |
| P,A | WO 2006/060363 A (ALCON INC) 8 June 2006 (2006-06-08) * the whole document * ----- | 1-10 | |

-/--

The present search report has been drawn up for all claims

| Place of search | Date of completion of the search | Examiner |
|---|---|---|
| Berlin | 29 March 2007 | KUEHNE, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 06 25 6136

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | WATSKY M A ET AL: "Predicting refractive alterations with hydrogel keratophakia." INVESTIGATIVE OPHTHALMOLOGY & VISUAL SCIENCE FEB 1985, vol. 26, no. 2, February 1985 (1985-02), pages 240-243, XP008077046 ISSN: 0146-0404 * the whole document * | | |

----- 

TECHNICAL FIELDS SEARCHED (IPC)

The present search report has been drawn up for all claims

| Place of search | Date of completion of the search | Examiner |
|---|---|---|
| Berlin | 29 March 2007 | KUEHNE, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 25 6136

This annex lists the patent family members relating to the patent documents cited in the above-mentioned European search report.
The members are as contained in the European Patent Office EDP file on
The European Patent Office is in no way liable for these particulars which are merely given for the purpose of information.

29-03-2007

| Patent document cited in search report | | Publication date | Patent family member(s) | | Publication date |
|---|---|---|---|---|---|
| EP 0308077 | A | 22-03-1989 | AU | 2214088 A | 16-03-1989 |
| | | | BR | 8804718 A | 18-04-1989 |
| | | | JP | 1195853 A | 07-08-1989 |
| | | | US | 5123921 A | 23-06-1992 |
| WO 03041616 | A | 22-05-2003 | US | 2005090895 A1 | 28-04-2005 |
| | | | US | 2003093083 A1 | 15-05-2003 |
| US 5716633 | A | 10-02-1998 | NONE | | |
| WO 03101341 | A | 11-12-2003 | AU | 2003243393 A1 | 19-12-2003 |
| | | | BR | 0305058 A | 09-11-2004 |
| | | | CA | 2485508 A1 | 11-12-2003 |
| | | | EP | 1549238 A2 | 06-07-2005 |
| | | | JP | 2005528165 T | 22-09-2005 |
| | | | MX | PA04012162 A | 19-04-2005 |
| | | | US | 2006189966 A1 | 24-08-2006 |
| WO 2006060363 | A | 08-06-2006 | US | 2006116762 A1 | 01-06-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WARSKY, M.** Predicting Refractive Alterations with Hydrogel Keratophakia. *Investigative Ophthalmology & Visual Science,* 1985, vol. 26 (2), 240-243